# EUROPEAN PATENT SPECIFICATION

(11) **EP 3 229 855 B1**
(45) Date of publication and mention of the grant of the patent: **08.05.2024**
(21) Application number: 15867142.0
(22) Date of filing: 09.12.2015
(51) Int. Cl.: A61L 31/08, A61L 31/16, A61K 38/17, A61L 27/54

(54) **NOVEL WOUND-HEALING-ENHANCING DEVICES**
NEUARTIGE WUNDHEILUNGSVERBESSERUNGSVORRICHTUNGEN
NOUVEAUX DISPOSITIFS FAVORISANT LA CICATRISATION

(30) Priority: 09.12.2014 US 201462089756 P
(43) Date of publication of application: 18.10.2017
(73) Proprietor: The Regents of the University of California, Oakland, CA 94607-5200 (US)
(72) Inventor: SOO, B. Chia, Beverly Hills, California 90211 (US); TING, Kang, Beverly Hills, California 90211 (US); ZHENG, Zhong, Van Nuys, California 91405 (US); WU, Benjamin, San Marino, California 91108 (US); ZHANG, Yulong, Los Angeles, California 90066 (US)
(74) Representative: Page White Farrer
(86) International application number: PCT/US2015/064826
(87) International publication number: WO 2016/094577

(56) References cited:
- WO-A1-2005/082430
- WO-A2-2009/105761
- WO-A2-2010/138637
- WO-A2-2011/005493
- WO-A2-2012/174280
- US-A1- 2013 330 397
- US-B2- 8 063 264

## Description

### FIELD OF THE INVENTION

The present invention relates to wound-healing-enhancing devices.

### BACKGROUND

Fibrotic tissue adhesion and scar formation around medical devices (including but not limited to suture, stents, and mesh) are major complaints in current medical practices. However, current available medical devices do not effectively prevent fibrogenesis and scarring.

Fibromodulin (FMOD) is a member of a family of small interstitial proteoglycans that also includes decorin, biglycan and lumican. The proteoglycans bind to other matrix - molecules and thereby help to stabilize the matrix. (Buckwalter et al., Instr. Course Lect 477-86 (1998); Iozzo et al., Biology of Excellullar Matrix 197-231 (2011)). Proteoglycan protein cores are structurally related and consist of a central region of leucine-rich repeats flanked by disulfide-bonded terminal domains. Fibromodulin has up to 4 keratin sulfate chains within its leucine rich domain. It has a wide tissue distribution and is most abundant in articular cartilage, tendon and ligament. It has been suggested that fibromodulin participates in the assembly of the extracellular matrix due to its ability to interact with type I and type II collagen fibrils and to inhibit fribrillogenesis *in vitro.* In addition SLRP have matricellular functions to influence the function of cells and determine cell fate by modulating the matrix, or binding the cell surface receptors, or indirectly through modulation of growth factors (Iozzo et al., Biology of Extracellular Matrix 197-231 (2011), Zheng et al. Biomaterials 5821-31 (2012)).

The embodiments described below address the above described problems.

WO 2012/174280 A2 discloses methods and compositions for treating or ameliorating a condition associated with increased or decreased myofibroblast activities.

WO 2010/138637 A2 discloses a fibromodulin (FMOD) peptide (FMOD-P), a composition and a formulation comprising a FMOD-P, optionally with a TGF-β isoform, or comprising FMOD with a TGF-β isoform. Also disclosed are methods of making and using the FMOD-P, composition, and formulation.

### SUMMARY OF THE INVENTION

One aspect of the present invention provides a biocompatible device according to claim 1.

In another aspect of the present invention, there is provided a method of fabricating a biocompatible device, according to claim 3.

Certain more specific aspects of the present invention are set out in the dependent claims.

### BRIEF DESCRIPTION OF THE DRAWINGS

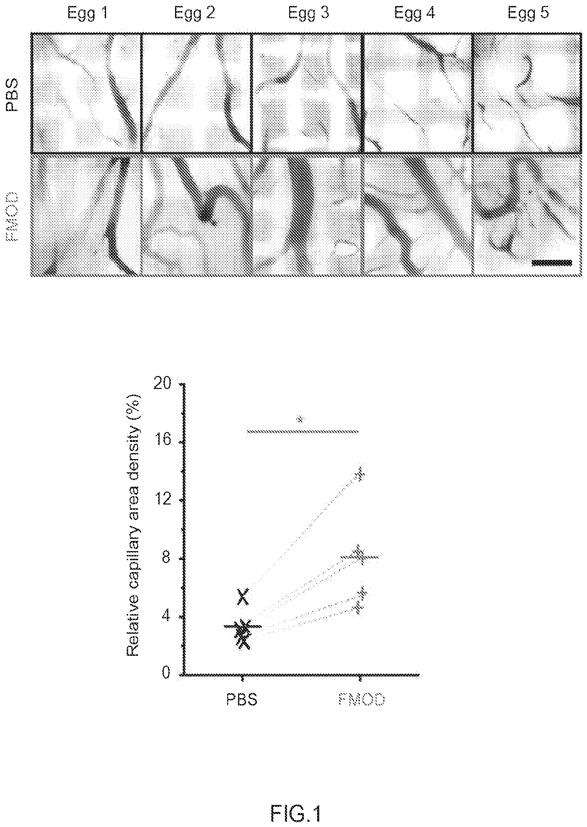

Figure 1 shows effects of FMOD on vascularization assessed by *in ovo* chick embryo chorioallantoic membrane (CAM) assay. Macroscopic photographs (above) and computerized quantitation (below) showed significantly increased more capillary generation on 30 µl of 2.0 mg/ml FMOD-treated CAMs than on phosphate buffered saline (PBS)-control groups. Significant differences compared by paired *t*-test *(P* < 0.05) are marked with asterisks (N = 5). Bar = 500 µm.

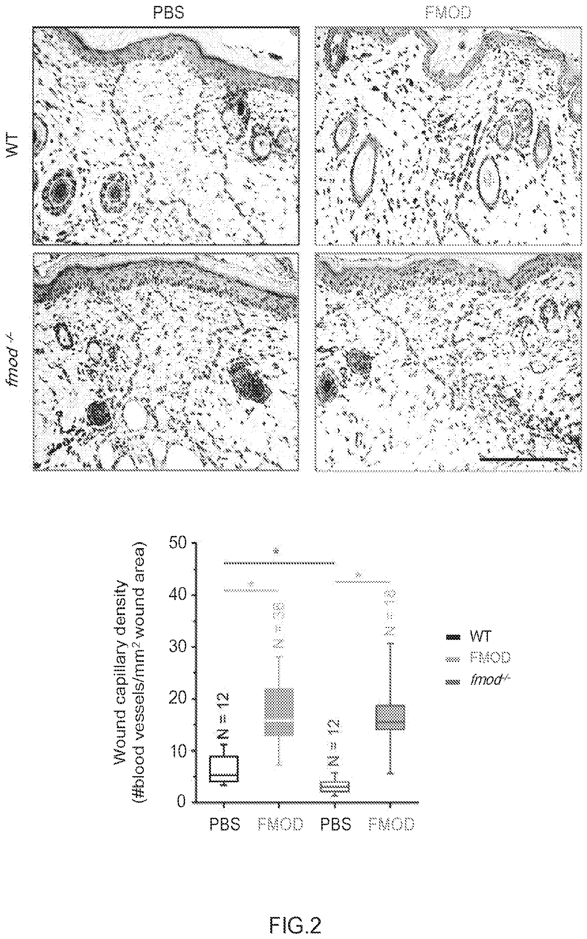

Figure 2 shows von Willebrand Factor (vWF) staining of adult mouse cutaneous wounds. Sections of PBS- treated wild-type (WT; above, left), FMOD-treated WT (above, right), PBS-treated *fmod*^{*-*/*-*} (center, left), FMOD-treated *fmod*^{-/-} (center, right) wounded mouse skin at day 14 post-injury, whose wound capillary density was quantitated (below). Wound areas are outlined by dashed lines, and blood vessels are indicated by red arrowheads. FMOD: 0.4 mg/ml x 50 µl/wounds. Significant differences compared by Mann-Whitney analysis (*P* < 0.05) are marked with asterisks: red asterisk indicates significance resulting from fmod knockout, and blue asterisks indicate significance resulting from FMOD administration. Bar = 200 µm.

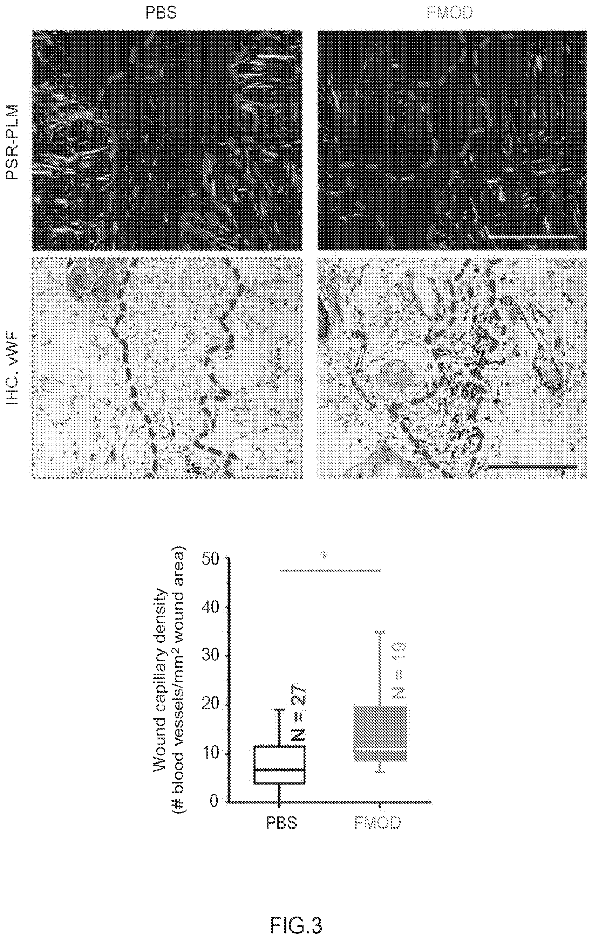

Figure 3 shows vWF staining of rat cutaneous wounds at day 14 post- injury. Picrosirius Red staining coupled polarized light microscopy (PSR-PLM) demonstrated the wound area (above; outlined by dashed lines), while the blood vessels were identified by IHC staining against vWF (center; red arrowheads) and were quantitated (below). Hematoxylin and eosin (H&E) staining of the identical wounds were shown in. FMOD: 0.4 mg/ml x 50 µl/wounds. Significant differences compared by Mann-Whitney analysis (P < 0.05) are marked with asterisks. Bar = 200 µm.

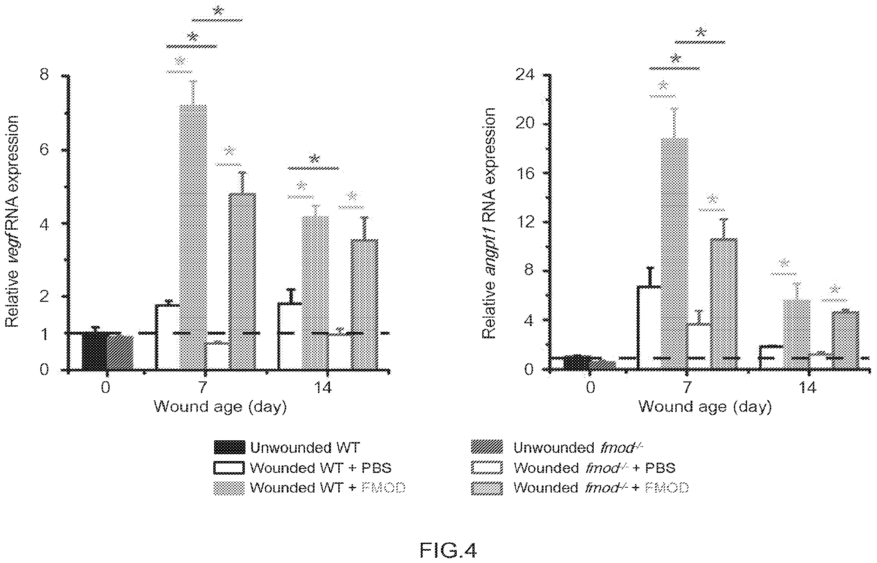

Figure 4 shows gene expression in adult WT and *fmod*^{*-*/*-*} mouse cutaneous wounds. Expression levels of *vegf* (left) and *angpt1* (right) were measured by real-time PCR and were normalized to uninjured adult WT skin tissue (dashed lines). FMOD: 0.4 mg/ml x 50 µl/wounds. Data are presented as mean ± SD (N = 3 different cDNA templates, each template underwent reverse-transcription from an RNA pool of 3 wounds harvested from 3 different animals, a total of 9 wounds from 9 animals per treatment were used). Significant differences compared by two-sample *t*-test *(P* < 0.05) are marked with asterisks: red asterisks indicate the significance from *fmod*^{*-*/}*⁻,* and blue asterisks indicate the significance that resulted from exogenous FMOD administration.

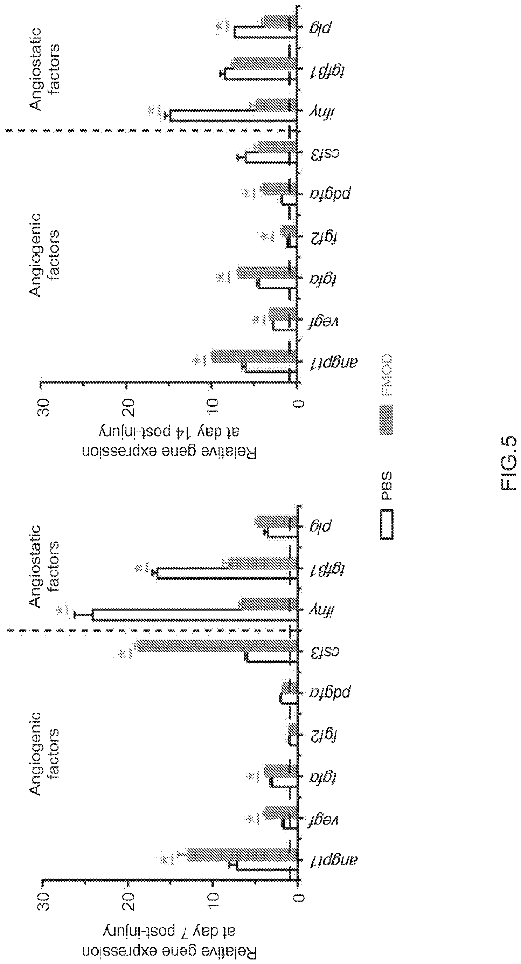

Figure 5 shows RT² PCR Array for angiogenic and angiostatic gene expression during adult rat cutaneous wound healing. Gene expression at day 7 (above) and 14 (below) post-injury are shown. Angiogenic genes include *angpt1, vegf, tgfα, fgf2, pdgfα,* and *csf3;* while angiostatic genes include *ifnγ, tgfβ1,* and *plg:* 0.4 mg/ml × 50 µl/wounds. Data are presented as mean ± SD (N = 3 different cDNA templates, each template underwent reverse-transcription from an RNA pool of 3 wounds harvested from 3 different animals, a total of 9 wounds from 9 animals per treatment were used) and normalized to uninjured rat skin tissue (dashed lines). Significant differences compared by two-sample *t*-test *(P* < 0.05) are marked with asterisks.

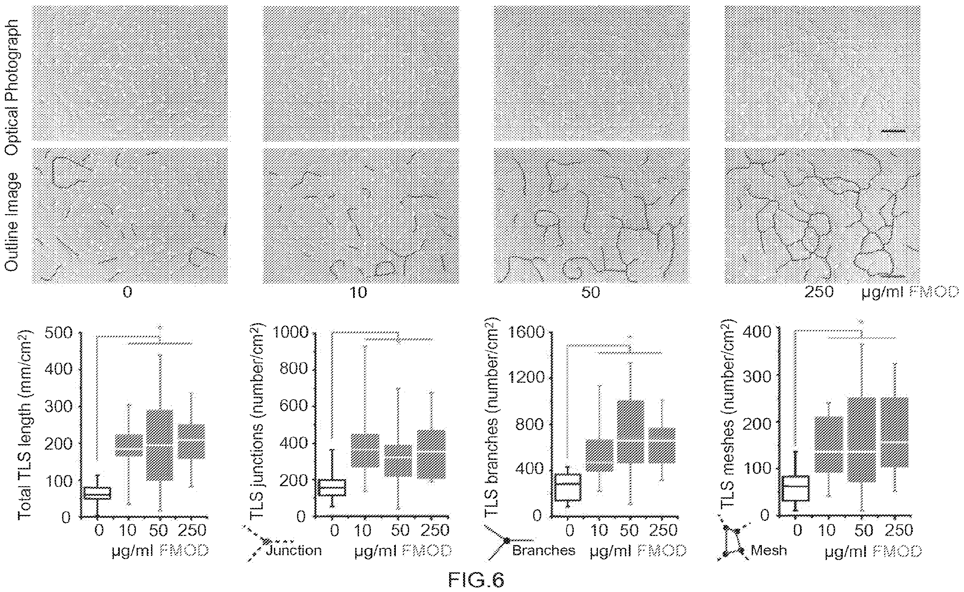

Figure 6 shows tube-like structures (TLSs) formation by human umbilical vein endothelial cells (HUVEC cells) on Geltrex^{®} matrix *in vitro.* Light microscopy of HUVEC cells spontaneously formed TLSs (outlined; above). Dimensional and topological parameters of the HUVEC TLS network were quantified (below). Significant differences compared by Mann-Whitney analysis (*P* < 0.05) are marked with asterisks (N = 16). Bar = 200 µm.

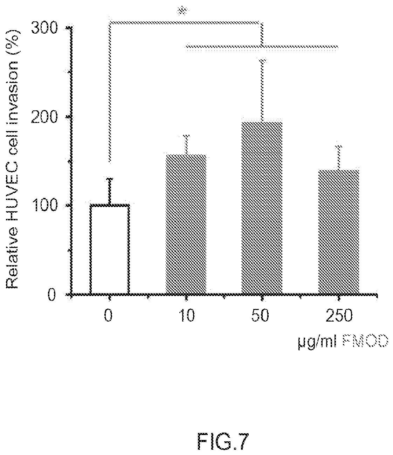

Figure 7 shows *in vitro* invasion assay of HUVEC cells. Data are presented as mean ± SD (N = 6) and normalized to non-FMOD PBS-treated control group.
   Significant differences compared by two-sample *t*-test *(P* < 0.05) are marked with asterisks. One-way ANOVA analysis revealed there is no significant difference between 10, 50, and 250 µg/ml FMOD groups.

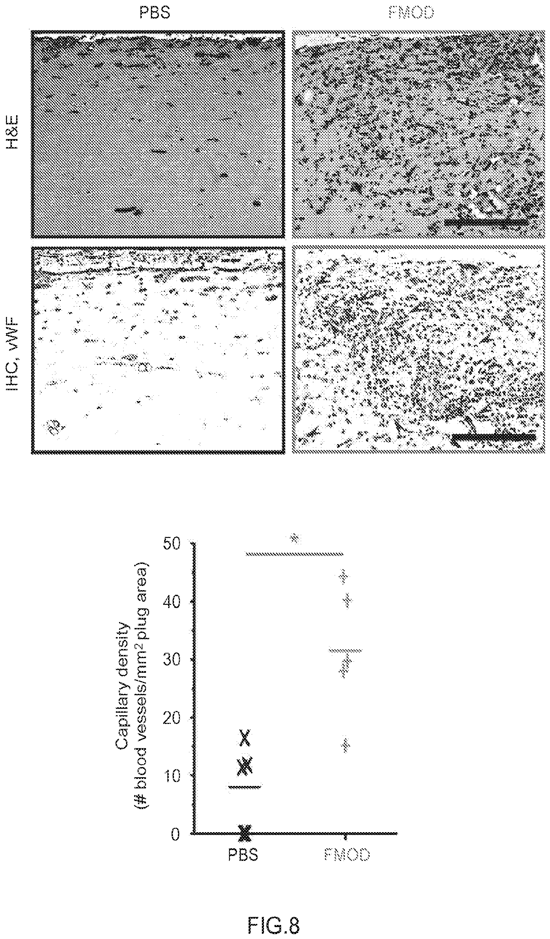

Figure 8 shows Matrigel^{™} plugs subcutaneously injected into the abdomen of adult 129/sv male mouse. H&E staining (about) is shown with IHC staining against vWF (center) which was used to identify and quantitate blood vessels (below). Blood vessels are indicated with red arrowheads. FMOD: 4.0 mg/ml x 400 µl/plug. Significant differences compared by Mann-Whitney analysis (*P* < 0.05) are marked with asterisks (N = 5). Bar = 200 µm.

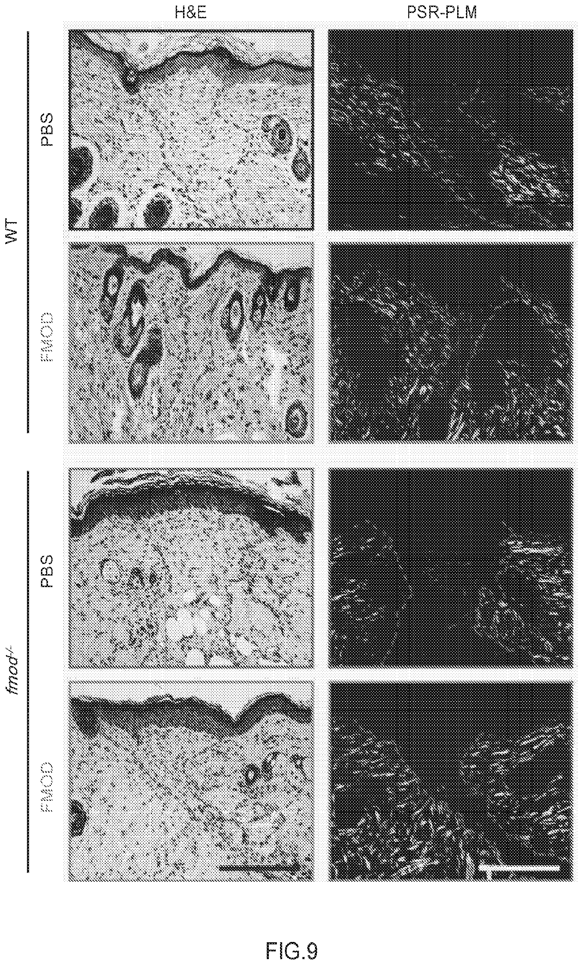

Figure 9 shows H&E staining and PSR-PLM demonstrate of adult mouse cutaneous wounds (outlined by dashed lines) at day 14 post-injury. FMOD: 0.4 mg/ml x 50 µl/wounds. Bar = 200 µm.

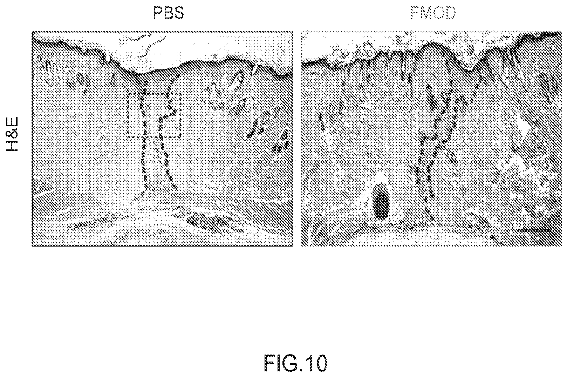

Figure 10 shows H&E staining of adult rat cutaneous wounds at day 14 post injury. The wound area was outlined by dashed lines), while IHC staining areas were outlined by dashed boxes. FMOD: 0.4 mg/ml x 50 µl/wounds. Bar = 400 µm.

### DETAILED DESCRIPTION

We have demonstrated that fibromodulin (FMOD) protein and its derived peptide can optimize wound healing by accelerating cell migration and contraction, minimizing fibrotic extracellular matrix (ECM) production and deposition, while increasing tensile strength. This procedure for protein/peptide-coating on various medical devices is different from our previous invention, which uses FMOD protein or its derived peptide directly as a wound-healing-enhancing therapeutic medicine.

### Definitions

As used herein, the term "fibromodulin polypeptide" refers to a polypeptide of SEQ ID NO. 1 (Genbank Accession No. NM 002023) or to a conservative substitution variant or fragment thereof that retains fibromodulin activity as that term is defined herein. It should be understood that the carbohydrate moieties of fibromodulin can be involved in fibromodulin pro-angiogenic activity, including, e.g., N-linked keratin sulfate chains. The leucine-rich repeats in the C-terminal domain of the fibromodulin polypeptide have been implicated in the binding of fibromodulin to type I collagen and can play a role in fibromodulin pro-angiogenic activity. See e.g., Kalamajski and Oldberg, (2007) J Biol Chem 282:26740-26745, which highlights the role of leucine-rich repeats in type I collagen binding. By "retaining fibromodulin activity" is meant that a polypeptide retains at least 50% of the fibromodulin activity of a polypeptide of SEQ ID NO. 1. Also encompassed by the term "fibromodulin polypeptide" are mammalian homologs of human fibromodulin and conservative substitution variants or fragments thereof that retain fibromodulin activity. In one aspect, such homologs or conservative variants thereof stimulate human endothelial cell growth and/or migration as measured, for example, as described herein. In some embodiments, the term fibromodulin polypeptide also includes the various peptides disclosed in U.S. patent application publication No. 20120171253.

The term "variant" as used herein refers to a polypeptide or nucleic acid that is "substantially similar" to a wild-type fibromodulin polypeptide or polynucleic acid. A molecule is said to be "substantially similar" to another molecule if both molecules have substantially similar structures (i.e., they are at least 50 % similar in amino acid sequence as determined by BLASTp alignment set at default parameters) and are substantially similar in at least one relevant function (e.g., effect on cell migration). A variant differs from the naturally occurring polypeptide or nucleic acid by one or more amino acid or nucleic acid deletions, additions, substitutions or side-chain modifications, yet retains one or more specific functions or biological activities of the naturally occurring molecule amino acid substitutions include alterations in which an amino acid is replaced with a different naturally-occurring or a non-conventional amino acid residue. Some substitutions can be classified as "conservative," in which case an amino acid residue contained in a polypeptide is replaced with another naturally occurring amino acid of similar character either in relation to polarity, side chain functionality or size. Substitutions encompassed by variants as described herein can also be "non-conservative," in which an amino acid residue which is present in a peptide is substituted with an amino acid having different properties (e.g., substituting a charged or hydrophobic amino acid with an uncharged or hydrophilic amino acid), or alternatively, in which a naturally-occurring amino acid is substituted with a non-conventional amino acid. Also encompassed within the term "variant," when used with reference to a polynucleotide or polypeptide, are variations in primary, secondary, or tertiary structure, as compared to a reference polynucleotide or polypeptide, respectively (e.g., as compared to a wild-type polynucleotide or polypeptide). Polynucleotide changes can result in amino acid substitutions, additions, deletions, fusions and truncations in the polypeptide encoded by the reference sequence. Variants can also include insertions, deletions or substitutions of amino acids, including insertions and substitutions of amino acids and other molecules) that do not normally occur in the peptide sequence that is the basis of the variant, including but not limited to insertion of ornithine which does not normally occur in human proteins.

The term "derivative" as used herein refers to peptides which have been chemically modified, for example by ubiquitination, labeling, PEGylation (derivatization with polyethylene glycol), glycosylation, protein fusion, or addition of other molecules. A molecule is also a "derivative" of another molecule when it contains additional chemical moieties not normally a part of the molecule. Such moieties can improve the molecule's solubility, absorption, biological half-life, etc. The moieties can alternatively decrease the toxicity of the molecule, or eliminate or tenuate an undesirable side effect of the molecule, etc. Moieties capable of mediating such effects are disclosed in Remington's Pharmaceutical Sciences, 18th edition, A. R. Gennaro, Ed., Mack Publ., Easton, Pa. (1990).

The term "functional" when used in conjunction with "derivative" or "variant" refers to polypeptides which possess a biological activity that is substantially similar to a biological activity of the entity or molecule of which it is a derivative or variant. By "substantially similar" in this context is meant that at least 50 % of the relevant or desired biological activity of a corresponding wild-type peptide is retained. In the instance of promotion of angiogenesis, for example, an activity retained would be promotion of endothelial cell migration; preferably the variant retains at least 60 %, at least 70 %, at least 80 %, at least 90 %, at least 95 %, at least 100 % or even higher (i.e., the variant or derivative has greater activity than the wild-type), e.g., at least 110%, at least 120%, or more compared to a measurable activity (i.e., promotion or inhibition of endothelial cell migration) of the wild-type polypeptide.

The term "wound-healing-enhancing agent" refers to an agent that is effective to produce a statistically significant, measurable improvement in wound healing of an injured tissue, e.g., reduction in fibrotic formation, e.g., reduction of scar formation, as compared with wound healing without using the wound-healing-enhancing agent of the present disclosure. As such, the term "wound-healing-enhancing effective amount" is an amount of an agent that is sufficient to produce a statistically significant, measurable improvement in wound healing of an injured tissue, e.g., reduction in fibrotic formation, e.g., reduction of scar formation, as compared with wound healing without using the wound-healing-enhancing agent of the present disclosure. As used herein, the term "wound-healing-enhancing effective amount" explicitly excludes an amount for forming reduced scarring in skin healing or cornea.

As used herein, the term "wound" includes an acute wounds as well as chronic wounds. Wound-healing includes healing of wounds in any tissue, e.g., healing fascia, tendon, etc. A mode of action of FMOD is to accelerate cell migration, cell contraction, and to minimize ECM deposition, while increasing tensile strength of the repair. The present invention has broad applications to everything aspect of wound repair including anti-fibrosis.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially" of refers to those elements required for a given embodiment. The term permits the presence of elements that do not materially affect the basic and novel or functional characteristic(s) of that embodiment of the invention.

The term "consisting" of refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth.

The term "carrier" includes any biocompatible material, which can be a metallic material or non-metallic materials. Examples of metallic materials includes titanium, zirconium, silver, gold, platinum, stainless steel, Cr-Co alloy, a Ti-Ni alloy, aluminum, magnesium, or any alloy formed thereof. Non-metallic materials include cement, ceramics, bioglass, natural polymer, synthetic polymer, for example. Some examples of polymer materials are described further below.

As used herein, the term "a wound-healing agent" refers to an agent that is commonly used in wound-healing.

As used herein, the term "body structure" refers to the mechanical structure of a device of invention provided herein.

### Biocompatible devices

The present disclosure provides a biocompatible device comprising a wound-healing-enhancing agent in a wound-healing-enhancing effective amount.

In some implementations of the disclosed device, optionally in combination with any or all of the various implementations disclosed herein, the biocompatible device further comprises a carrier, wherein the wound-healing-enhancing agent is included in the carrier.

In some implementations of the disclosed device, optionally in combination with any or all of the various implementations disclosed herein, the biocompatible device further comprises a coating comprising a polymeric material.

In some implementations of the disclosed device, optionally in combination with any or all of the various implementations disclosed herein, the biocompatible device further comprises a wound-healing agent. Such wound-healing agent is different from the wound-healing-enhancing agent of disclosure. Examples of such wound-healing agent can be, for example, an antimicrobial agent, an anti-inflammatory agent, or an anti-fibrotic agent.

In some implementations of the disclosed device, optionally in combination with any or all of the various implementations disclosed herein, the polymeric material forms a top layer on top of the layer of a wound-healing-enhancing effective amount of a wound-healing-enhancing agent.

In some implementations of the disclosed device, optionally in combination with any or all of the various implementations disclosed herein, the carrier is a matrix material admixed with or encapsulating a wound-healing-enhancing effective amount of a wound-healing-enhancing agent.

In some implementations of the disclosed device, optionally in combination with any or all of the various implementations disclosed herein, the carrier comprises microparticles/nanoparticles, wherein the wound-healing-enhancing agent is encapsulated into microparticles/nanoparticles.

In some implementations of the disclosed device, optionally in combination with any or all of the various implementations disclosed herein, the microparticles/nanoparticles are admixed or encapsulated into a polymeric material matrix.

In connection with the above various carrier, matrix, or coating configurations, the top layer or the matrix material can be used along or in combination to provide controlled release of the wound-healing-enhancing agent from the biocompatible device. In the case where the coated device includes no polymeric material, the release of the wound-healing-enhancing agent from the biocompatible device is burst release.

In some implementations of the disclosed device, optionally in combination with any or all of the various implementations disclosed herein, the wound-healing-enhancing agent is fibromodulin (FMOD), a FMOD polypeptide, a FMOD peptide, or a variant or analog or derivative thereof.

The biocompatible device can be any device for use where a disorder can be treated or alleviated by implanting the device in a subject (e.g., a human patient). Examples of such biocompatible devices include, but not limited to, implanted medical devices (e.g, mesh, anti-adhesion devices, nerve or vascular conduits, breast implants, tissue expanders, pacemakers, defibrillators, neurostimulators, or other electrical devices), percutaneously delivered devices (e.g., stents), wound closure devices (e.g., sutures, staples), wound management coverings (tapes, membranes for guided tissue regeneration) and tissue engineering scaffolds for which scar formation must be avoided (e.g. orbital wall reconstruction - bone formation must occur without scarring of the soft tissues) to avoid undesired surgery-induced fibrotic adhesion and scar formation or gliosis (scar in central nervous system) in all operated on tissues on organ systems (e.g., brain, heart, lungs, liver, intestine, blood vessels, nerves, muscle, tendon, eye, inner ear, sinus, etc.) using all available approaches (e.g., open surgery, endoscopic surgery, minimally invasive, percutaneous, etc.)

In some implementations, the biocompatible device can be cosmetic implants. Such cosmetic implants include, e.g., a breast implant, pectoral implant, chin implant, or malar implant.

In some implementations, the biocompatible device specifically excludes such devices disclosed in WO2004053101 A3 or WO2009135135 A3 in connection with skin wound healing or cornea wound healing.

Some other examples of the biocompatible devices are stents, such as protein-eluting biodegradable polymer stents on anastomotic wound healing after biliary reconstruction, or coronary stents, which are implanted in narrowed coronary arteries during surgery.

### Fibromodulin

Fibromodulin (FMOD), also called SLRR2E, is a member of a family of small interstitial proteoglycans. The protein is 59 kDa with leucine-rich repeats flanked by disulfide-bonded terminal domains, possessing up to 4 keratan sulfate chains (Takahashi, T., Cho, H. I., Kublin, C. L. & Cintron, C. (1993) J Histochem Cytochem 41:1447-57). Fibromodulin exhibits a wide tissue distribution with the highest concentration found in articular cartilage, tendon, and ligament. The subcellular location of fibromodulin is within the cytosolic proteins with a secretory sequence but no trans-membrane or extracellular domain.

While it is not wished to indicate that such activity is critical to the pro-angiogenic activity of fibromodulin, several activities of fibromodulin are worth noting here. A characteristic feature of this protein is its participation in the assembly of the extracellular matrix by virtue of its ability to interact with type I, type II and XII collagen fibrils to form collagen fibrils network (Hedbom, E. & Heinegard, D. (1993) J Biol Chem 268: 27307-12; Font, B., Eichenberger, D., Goldschmidt, D., Boutillon, M. M. & Hulmes, D. J. (1998) Eur J Biochem 254:580-7) and to inhibit fibrillogenesis *in vitro* (Antonsson, P., Heinegard, D. & Oldberg, A. (1991) J Biol Chem 266:16859-61; Hedlund, H., Mengarelli-Widholm, S., Heinegard, D., Reinholt, F. P. & Svensson, 0. (1994) Matrix Biol 14:227-32; Ezura, Y., Chakravarti, S., Oldberg, A., Chervoneva, I. & Birk, D. E. (2000) J Cell Biol 151:779-88; Gori, F., Schipani, E. & Demay, M. B. (2001) J Cell Biochem 82:46-57; Ameye, L. et al. (2002) Faseb J16: 673-80; Ameye, L. & Young, M. F. (2002) Glycobiology 12:107R-16R; Chakravarti, S. (2002) Glycoconj J19:287-93*)* FMOD interaction with transforming growth factor (TGF)-β, a key profibrotic cytokine, is considered to enhance the retention of this growth factor within the extracellular matrix (ECM), thus regulating TGF-β local action (Burton-Wurster, N. et al. (2003) Osteoarthritis Cartilage 11:167-76*;* San Martin, S. et al. (2003) Reproduction 125:585-95; Fukushima, D., Butzow, R., Hildebrand, A. & Ruoslahti, E. (1993) J Biol Chem 268:22710-5; Hildebrand, A. et al. (1994) Biochem J302 (Pt 2):527-34). The protein is involved in a variety of adhesion processes of connective tissue, and with immunoglobulins activating both the classical and the alternative pathways of complement. Further studies revealed that fibromodulin binds directly to the globular heads of Clq, leading to activation of Cl. Fibromodulin also binds complement inhibitor factor H (Sjoberg, A. P. et al. (2007) J Biol Chem 282:10894-900; Sjoberg, A., Onnerfjord, P., Morgelin, M., Heinegard, D. & Blom, A. M. (2005) J Biol Chem 280:32301-8).

The fibromodulin gene has been found to be an overexpressed gene in B-cell chronic lymphocytic leukemia and chronic lymphocytic leukemia (CLL). It may serve as a potential tumor-associated antigen (TAA) in CLL (Mayr, C. et al. (2005) Blood 105:1566-73; Mayr, C. et al. (2005) Blood 106:3223-6). The amino acid sequences of human and bovine, rat and murine fibromodulin show an overall homology of 90 %, allowing for close translation between human and murine experimental models (Antonsson, P., Heinegard, D. & Oldberg, A. (1993) Biochim Biophys Acta 1174:204-6).

### Fibromodulin Activity Enhancers

Essentially any agent that enhances fibromodulin activity, as that term is defined herein, can be used with the methods described herein. It is preferred, however, that an enhancer of fibromodulin activity is specific, or substantially specific, for fibromodulin activity promotion. Further, it is noted that fibromodulin activity can be enhanced by agents that specifically promotes the expression of the fibromodulin proteoglycan.

It will be understood by one of skill in the art that the method and composition provided herein can be used to impart enhancing wound healing and/or any other advantageous property to any device that is used as a medical implant. Devices provided herein include medical implants, scaffolds and/or medical instruments. Exemplary medical implants include but are not limited to sutures, stents, balloons, valves, pins, rods, screws, discs, and plates. Exemplary medical implants include but are not limited to an artificial replacement of a body part such as a hip, a joint, etc.

The implant can be one of cosmetic implants, such as but not limited to breast implants, pectoral implants, chin implants, malar implants, et al.

In some implementations, the devices include a biocompatible intervertebral device (e.g., a cervical fusion device).

Devices disclosed herein include those associated with dental surgeries, including but not limited to a disc, a bridge, a retainer clip, a screw, a housing, a bone graft, and/or a crown.

Devices disclosed herein include those associated with orthopedic surgeries, including, for example, intramedullary rods, temporary and permanent pins and implants, bone plates, bone screws and pins, and combinations thereof.

Additional information on biocompatible medical devices and osteoinductive materials can be found, for example, in United States Patent Publication No. 2009/0012620 by Youssef J., et al. and entitled "Biocompatible Cervical Fusion Device;" United States Patent No. 5,348,026 to Davidson and entitled "Osteoinductive Bone Screw;" Barradas A. et al, 2011, "Osteoinductive Biomaterials: Current Knowledge of Properties, Experimental Models and Biological Mechanisms," European Cells and Materials 21 :407-429; United States Patent No. 7,485,617 to Pohl J. et al. and entitled "Osteoinductive Materials," United States Patent Publication No. 2011/0022180 by Melkent A., et al. and entitled "Implantable Medical Devices;" United States Patent Publication No. 2005/0010304 by Jamali, A. and entitled "Device and Method for Reconstruction of Osseous Skeletal Defects;" United States Patent Publication No. 2010/0036502 by Svrluga R. et al. and entitled "Medical Device for Bone Implant and Method for Producing Such Device;" United States Patent No. 5,672,177 to E. Seldin and entitled "Implantable Bone Distraction Device;" and United States Patent No. 4,611,597 to W. Kraus and entitled "Implantable Device for the Stimulation of Bone Growth".

### Fibromodulin Polypeptides

A fibromodulin polypeptide or a portion thereof functional to promote angiogenesis can be administered to an individual in need thereof. In one approach, a soluble fibromodulin polypeptide, produced, for example, in cultured cells bearing a recombinant fibromodulin expression vector can be administered to the individual. The fibromodulin polypeptide or portion thereof will generally be administered intravenously. This approach rapidly delivers the protein throughout the system and maximizes the chance that the protein is intact when delivered. Alternatively, other routes of therapeutic protein administration are contemplated, such as by inhalation. Technologies for the administration of agents, including protein agents, as aerosols are well known and continue to advance. Alternatively, the polypeptide agent can be formulated for topical delivery, including, for example, preparation in liposomes. Further contemplated are, for example, trans-dermal administration, and rectal or vaginal administration. Further options for the delivery of fibromodulin polypeptides as described herein are discussed in the section "Pharmaceutical Compositions" herein below.

Vectors for transduction of a fibromodulin-encoding sequence are well known in the art. While overexpression using a strong non-specific promoter, such as a CMV promoter, can be used, it can be helpful to include a tissue- or cell-type-specific promoter on the expression construct for example, the use of a skeletal muscle-specific promoter or other cell-type-specific promoter can be advantageous, depending upon what cell type is used as a host. Further, treatment can include the administration of viral vectors that drive the expression of fibromodulin polypeptides in infected host cells. Viral vectors are well known to those skilled in the art.

These vectors are readily adapted for use in the methods of the present disclosure. By the appropriate manipulation using recombinant DNA/molecular biology techniques to insert an operatively linked fibromodulin encoding nucleic acid segment into the selected expression/delivery vector, many equivalent vectors for the practice of the methods described herein can be generated. It will be appreciated by those of skill in the art that cloned genes readily can be manipulated to alter the amino acid sequence of a protein.

The cloned gene for fibromodulin can be manipulated by a variety of well-known techniques for *in vitro* mutagenesis, among others, to produce variants of the naturally occurring human protein, herein referred to as muteins or variants or mutants of fibromodulin, which may be used in accordance with the methods and compositions described herein. The variation in primary structure of muteins of fibromodulin useful in the invention, for instance, may include deletions, additions and substitutions. The substitutions may be conservative or non-conservative. The differences between the natural protein and the mutein generally conserve desired properties, mitigate or eliminate undesired properties and add desired or new properties. The fibromodulin polypeptide can also be a fusion polypeptide, fused, for example, to a polypeptide that targets the product to a desired location, or, for example, a tag that facilitates its purification, if so desired. Fusion to a polypeptide sequence that increases the stability of the fibromodulin polypeptide is also contemplated. For example, fusion to a serum protein, e.g., serum albumin, can increase the circulating half-life of a fibromodulin polypeptide. Tags and fusion partners can be designed to be cleavable, if so desired. Another modification specifically contemplated is attachment, e.g., covalent attachment, to a polymer. In one aspect, polymers such as polyethylene glycol (PEG) or methoxypolyethylene glycol (mPEG) can increase the *in vivo* half-life of proteins to which they are conjugated. Methods of PEGylation of polypeptide agents are well known to those skilled in the art, as are considerations of, for example, how large a PEG polymer to use. In another aspect, biodegradable or absorbable polymers can provide extended, often localized, release of polypeptide agents. Such synthetic bioabsorbable, biocompatible polymers, which may release proteins over several weeks or months can include, for example, poly-α-hydroxy acids (e.g. polylactides, polyglycolides and their copolymers), polyanhydrides, polyorthoesters, segmented block copolymers of polyethylene glycol and polybutylene terephtalate (Polyactive^{™}), tyrosine derivative polymers or poly(ester-amides). Suitable bioabsorbable polymers to be used in manufacturing of drug delivery materials and implants are discussed e.g. in U.S. Pat. Nos. 4,968,317; 5,618,563, among others, and in "Biomedical Polymers" edited by S. W. Shalaby, Carl Hanser Verlag, Munich, Vienna, New York, 1994 and in many references cited in the above publications. The particular bioabsorbable polymer that should be selected will depend upon the particular patient that is being treated.

### Polymeric Materials

The anti-fibrotic coating comprises a polymeric material. Exemplary polymeric material that can be used here include but are not limited to a biocompatible or bioabsorbable polymer that is one or more of poly(_{DL}-lactide), poly(_{L}-lactide), poly(_{L}-lactide), poly(_{L}-lactide-*co*-_{D,L}-lactide), polymandelide, polyglycolide, poly(lactide-*co*-glycolide), poly(_{D,L}-lactide-*co-*glycolide), poly(_{L}-lactide-*co*-glycolide), poly(ester amide), poly(ortho esters), poly(glycolic acid-*co*-trimethylene carbonate), poly(_{D,L}- lactide-*co*-trimethylene carbonate), poly(trimethylene carbonate), poly(lactide-*co*- caprolactone), poly(glycolide-*co*-caprolactone), poly(tyrosine ester), polyanhydride, derivatives thereof. In some embodiments, the polymeric material comprises a combination of these polymers.

In some embodiments, the polymeric material comprises poly(_{D,L}-lactide- *co-*glycolide). In some embodiments, the polymeric material comprises poly(_{D,L}-lactide). In some embodiments, the polymeric material comprises poly(_{L}-lactide). Additional exemplary polymers include but are not limited to poly(_{D}-lactide) (PDLA), polymandelide (PM), polyglycolide (PGA), poly(_{L}-lactide-*co*-_{D,L}-lactide) (PLDLA), poly(_{D,L}-lactide) (PDLLA), poly(_{D,L}-lactide-*co*-glycolide) (PLGA) and poly(_{L}- lactide-*co*-glycolide) (PLLGA). With respect to PLLGA, the stent scaffolding can be made from PLLGA with a mole% of GA between 5-15 mol%. The PLLGA can have a mole% of (LA:GA) of 85: 15 (or a range of 82: 18 to 88: 12), 95:5 (or a range of 93:7 to 97:3), or commercially available PLLGA products identified as being 85: 15 or 95:5 PLLGA. The examples provided above are not the only polymers that may be used. Many other examples can be provided, such as those found in Polymeric Biomaterials, second edition, edited by Severian Dumitriu; chapter 4.

In some embodiments, polymers that are more flexible or that have a lower modulus that those mentioned above may also be used. Exemplary lower modulus bioabsorbable polymers include, polycaprolactone (PCL), poly(trimethylene carbonate) (PTMC), polydioxanone (PDO), poly(3-hydrobutyrate) (PHB), poly(4-hydroxybutyrate) (P4HB), poly(hydroxyalkanoate) (PHA), and poly(butylene succinate), and blends and copolymers thereof.

In exemplary embodiments, higher modulus polymers such as PLLA or PLLGA may be blended with lower modulus polymers or copolymers with PLLA or PLGA. The blended lower modulus polymers result in a blend that has a higher fracture toughness than the high modulus polymer. Exemplary low modulus copolymers include poly(_{L}-lactide)-b-polycaprolactone (PLLA-*b*-PCL) or poly(_{L}-lactide)-co-polycaprolactone (PLLA-co-PCL). The composition of a blend can include 1-5 wt% of low modulus polymer.

More exemplary polymers include but are not limited to at least partially alkylated polyethyleneimine (PEI); at least partially alkylated poly(lysine); at least partially alkylated polyomithine; at least partially alkylated poly(amido amine), at least partially alkylated homo- and co-polymers of vinylamine; at least partially alkylated acrylate containing aminogroups, copolymers of vinylamine containing aminogroups with hydrophobic monomers, copolymers of acrylate containing aminogroups with hydrophobic monomers, and amino containing natural and modified polysaccharides, polyacrylates, polymethacryates, polyureas, polyurethanes, polyolefins, polyvinylhalides, polyvinylidenehalides, polyvinyl ethers, polyvinylaromatics, polyvinylesters, polyacrylonitriles, alkyd resins, polysiloxanes and epoxy resins, and mixtures thereof. Additional examples of biocompatible biodegradable polymers include, without limitation, polycaprolactone, poly(_{L}-lactide), poly(_{D,L}-lactide), poly(_{D,L}-lactide-co- PEG) block copolymers, poly(_{D,L}-lactide-*co*-trimethylene carbonate), poly(lactide-*co-* glycolide), polydioxanone (PDS), polyorthoester, polyanhydride, poly(glycolic acid-*co-*trimethylene carbonate), polyphosphoester, polyphosphoester urethane, poly(amino acids), polycyanoacrylates, poly(trimethylene carbonate), poly(iminocarbonate), polycarbonates, polyurethanes, polyalkylene oxalates, polyphosphazenes, PHA-PEG, and combinations thereof. The PHA may include poly(α-hydroxyacids), poly(β-hydroxyacid) such as poly(3-hydroxybutyrate) (PHB), poly(3-hydroxybutyrate-*co*-valerate) (PHBV), poly(3-hydroxyproprionate) (PHP), poly(3-hydroxyhexanoate) (PHH), or poly(4-hydroxyacid) such as poly(4-hydroxybutyrate), poly(4-hydroxyvalerate), poly(4-hydroxyhexanoate), polyhydroxyvalerate, poly(tyrosine carbonates), poly(tyrosine arylates), poly(ester amide), polyhydroxyalkanoates (PHA), poly(3-hydroxyalkanoates) such as poly(3-hydroxypropanoate), poly(3-hydroxybutyrate), poly(3-hydroxyvalerate), poly(3-ydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(_{D,L}-lactide), poly(_{L}-lactide), polyglycolide, poly(_{D,L}-lactide-*co*-glycolide), poly(_{L}-lactide-*co*-glycolide), polycaprolactone, poly(lactide-*co*-caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-*co*-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), polypropylene fumarate), poly(n-butyl methacrylate), poly(sec- butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), polypropylene oxide), poly(ether ester), polyalkylene oxalates, phosphoryl choline containing polymer, choline, poly(aspirin), polymers and copolymers of hydroxyl bearing monomers such as 2-hydroxyethyl methacrylate (HEMA), hydroxypropyl methacrylate (HPMA), hydroxypropylmethacrylamide, PEG acrylate (PEGA), PEG methacrylate, methacrylate polymers containing 2-methacryloyloxyethyl-phosphorylcholine (MPC) and n-vinyl pyrrolidone (VP), carboxylic acid bearing monomers such as methacrylic acid (MA), acrylic acid (AA), alkoxymethacrylate, alkoxy acrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate), MED610, poly(methyl methacrylate)-PEG (PMMA-PEG), polydimethylsiloxane-co-PEG (PDMS-PEG), poly(vinylidene fiuoride)-PEG (PVDF-PEG), PLURONIC^{™} surfactants (polypropylene oxide-co-polyethylene glycol), poly(tetramethylene glycol), hydroxy functional poly( vinyl pyrrolidone), biomolecules such as collagen, chitosan, alginate, fibrin, fibrinogen, cellulose, starch, dextran, dextrin, hyaluronic acid, fragments and derivatives of hyaluronic acid, heparin, fragments and derivatives of heparin, glycosamino glycan (GAG), GAG derivatives, polysaccharide, elastin, elastin protein mimetics, or combinations thereof.

In some embodiments, polyethylene is used to construct at least a portion of the device. For example, polyethylene can be used in an orthopedic implant on a surface that is designed to contact another implant, as such in a joint or hip replacement. Polyethylene is very durable when it comes into contact with other materials. When a metal implant moves on a polyethylene surface, as it does in most joint replacements, the contact is very smooth and the amount of wear is minimal. Patients who are younger or more active may benefit from polyethylene with even more resistance to wear. This can be accomplished through a process called crosslinking, which creates stronger bonds between the elements that make up the polyethylene. The appropriate amount of crosslinking depends on the type of implant. For example, the surface of a hip implant may require a different degree of crosslinking than the surface of a knee implant.

Additional examples of polymeric materials can be found, for example, in US Pat. No. 6,127,448 to Domb, entitled "Biocompatible Polymeric Coating Material;" US Pat. Pub. No. 2004/0148016 by Klein and Brazil, entitled "Biocompatible Medical Device Coatings;" US Pat. Pub. No. 2009/0169714 by Burghard et al*,* entitled "Biocompatible Coatings for Medical Devices;" US Pat. No. 6,406,792 to Briquet et al*,* entitled "Biocompatible Coatings;" US Pat. Pub. No. 2008/0003256 by Martens et al*,* entitled "Biocompatible Coating of Medical Devices;".

In some embodiments, a portion of or the entire device is formed by one or more the aforementioned polymeric materials provided herein. In some embodiments, the polymeric material used to form the device further comprises an antimicrobial agent such that the antimicrobial agent is embedded as a part of the device itself. In some embodiments, a biomedical material such as titanium, silicone or apatite is used to modify the surface of the device such that the device is biocompatible and does not trigger adverse reactions in a patient (e.g., a recipient of an implant).

In some embodiments, a portion of or the entire device is made from a metal material. Exemplary metal materials include but are not limited to stainless steel, chromium, a cobalt-chromium alloy, tantalum, titanium, a titanium alloy and combinations thereof.

Stainless steel is a very strong alloy, and is most often used in implants that are intended to help repair fractures, such as bone plates, bone screws, pins, and rods. Stainless steel is made mostly of iron, with other metals such as chromium or molybdenum added to make it more resistant to corrosion. There are many different types of stainless steel. The stainless steels used in orthopedic implants are designed to resist the normal chemicals found in the human body. Cobalt-chromium alloys are also strong, hard, biocompatible, and corrosion resistant. These alloys are used in a variety of joint replacement implants, as well as some fracture repair implants, that require a long service life. While cobalt-chromium alloys contain mostly cobalt and chromium, they also include other metals, such as molybdenum, to increase their strength. Titanium alloys are considered to be biocompatible. They are the most flexible of all orthopedic alloys. They are also lighter weight than most other orthopedic alloys. Consisting mostly of titanium, they also contain varying degrees of other metals, such as aluminum and vanadium. Pure titanium may also be used in some implants where high strength is not required. It is used, for example, to make fiber metal, which is a layer of metal fibers bonded to the surface of an implant to allow the bone to grow into the implant, or cement to flow into the implant, for a better grip. Tantalum is a pure metal with excellent physical and biological characteristics. It is flexible, corrosion resistant, and biocompatible.

### Method of Fabrication

The coated biocompatible device can be fabricated by forming a coating comprising a wound-healing-enhancing effective amount of a wound-healing-enhancing agent. In some embodiments, the wound-healing-enhancing agent can be formed as a layer on the biocompatible device. The wound-healing-enhancing agent is FMOD, FMOD polypeptide, FMOD peptide, or a variant or a derivative thereof such that the biocompatible device comprises a coating comprising a wound-healing-enhancing effective amount of FMOD, FMOD polypeptide, FMOD peptide, or a variant or a derivative thereof.

The coating further comprises a polymer material. The polymeric material can be a top layer formed on top of the layer of the wound-healing-enhancing agent. In some other embodiments, the polymeric material can be a matrix material admixed with or encapsulating the wound-healing-enhancing agent. The top layer or the matrix material can be used alone or in combination to provide controlled release of the wound-healing-enhancing agent from the biocompatible device. In the case where the coated device includes no polymeric material, the release of the wound-healing-enhancing agent from the biocompatible device is burst release.

In some embodiments, the coating can further comprise a polymer material matrix and encapsulated nanoparticles/microparticles. The polymeric material can be a top layer and as matrix encapsulating nanoparticles/mciroparticles containing the wound-healing-enhancing agent. In some other embodiments, the polymeric material can be a matrix material admixed with or encapsulating the nanoparticles/mciroparticles. The top layer or the matrix material can be used alone or in combination with particles to provide sustained release of the wound-healing-enhancing agent from the biocompatible device. In the case the nanoparticles/microparticles could be prepared by any material include but are not limited to natural polymers, synthetic polymers, inorganic chemicals, at least partially dextran, chitosan, collagen, silica, calcium phosphate, poly(_{D,L}-lactide), poly(_{L}-lactide), polyglycolide, poly(_{D,L}-lactide-co- glycolide), poly(_{L}-lactide-*co*-glycolide), polycaprolactone, poly(lactide-*co-*caprolactone), poly(glycolide-*co*-caprolactone), poly(dioxanone), poly(ortho esters), poly(anhydrides), poly(tyrosine carbonates) and derivatives thereof,
Methods of coating a device are well documented in the art. Examples of such methods include dip coating or spray coating using a solution or suspension, which solution or suspension comprising the wound-healing-enhancing agent disclosed herein, the polymeric material disclosed herein, alone or in combination.

### Method of Use

The wound-healing-enhancing biocompatible device can be used to treat, ameliorate, or improve any disorder in a subject that can be treated, ameliorated, or improved by a biocompatible device, which is well documented. Generally, the method comprises implanting the wound-healing-enhancing biocompatible device of invention in a subject in need thereof (e.g., a cardiovascular patient). Examples of such disorders include but not limited to cardiovascular diseases or conditions, orthopedic conditions or disorders, and diabetics.

### Examples

The following Examples are useful for understanding certain features of the invention, but are not in accordance with the present claims.

### Example 1: Fibromodulin enhances vascularization during cutaneous wound healing

### Summary

*Methods: In vivo* angiogenic effects of FMOD were assessed by a chick embryo chorioallantoic membrane (CAM) assay, a Matrigel^{™} plug implant assay, and rodent primary closure wound models. *In vitro* angiogenic effects of FMOD were recorded by cell invasion and dimensional and topological parameters of human umbilical vein endothelial cells (HUVECs).

*Results:* We provided evidence that FMOD significantly enhanced vascularization: firstly, FMOD boosted blood vessel formation on the CAM; secondly, FMOD markedly stimulated capillary infiltration into Matrigel^{™} plugs subcutaneously implanted in adult mice; and lastly, FMOD robustly promoted angiogenesis in multiple adult rodent cutaneous wound models. Furthermore, FMOD administration restored the vascularity of *fmod*^{*-*/*-*} mouse wounds. In support of this, FMOD endorsed an angiogenesis-favored microenvironment in adult rodent wounds not only by upregulating angiogenic genes, but also by downregulating angiostatic genes. Additionally, FMOD significantly enhanced HUVEC invasion and tube-like structure formation *in vitro.*

*Conclusion:* Altogether, we demonstrated that, in addition to reducing scar formation, FMOD also promotes angiogenesis. Since blood vessels organize and regulate wound healing, its potent angiogenic properties will further expand the clinical application of FMOD for cutaneous healing of poorly vascularized wounds.

### Introduction

Cutaneous wound healing is a natural response involving a complex cascade of cellular events to generate resurfacing, reconstitution, and restoration of tensile strength of injured skin. Unfortunately, the reasoning behind the failure of some cutaneous wounds to heal is still poorly understood due to the fact that wound healing is a complex, multifaceted process (1, 2). A fundamental problem of retarded wound healing is lack of a functional extracellular matrix (ECM) to stimulate, direct, and coordinate healing. For instance, deficiency of a single ECM molecule, fibromodulin (FMOD), in an adult mouse cutaneous wound model resulted in delayed dermal fibroblast migration, delayed granulation tissue formation, delayed wound closure, and subsequently increased scarring in an adult mouse cutaneous wound model (3). FMOD is a broadly distributed small leucine-rich proteoglycan (SLRP), which regulates ECM assembly, organization, and degradation via binding with collagens (4-10). FMOD plays an essential role in cell fate determination and fetal scarless wound healing (5, 11-14). In addition, our previous studies have demonstrated that FMOD controls significant aspects of adult cutaneous wound healing. Compared to their wild-type (WT) counterparts, FMOD-null (*fmod*^{*-*/*-*}) mice have reduced fibronectin deposition, unorganized collagen architecture, altered transforming growth factor (TGF)-β signaling, and reduced dermal fibroblast infiltration followed by impeded angiogenesis (3, 4, 16). On the other hand, FMOD administration in both adenoviral and protein forms reduced scar formation in adult cutaneous wounds (17, 18). Specifically, we have demonstrated that FMOD significantly promoted fibroblast migration into the wound area, aiding timely wound closure and reduced scar formation (3, 16, 19). Because newly generated blood vessels provide nutrients to support active cells, promote granulation tissue formation, and facilitate clearance of debris (20-22), wound healing cannot occur without angiogenesis, a process of neovascular formation by endothelial cells (ECs). Our previous studies revealed that retarded *fmod*^{*-*/}*⁻mouse* wound healing is associated with markedly reduced blood vessel regeneration (3), suggesting a direct relationship between FMOD and angiogenesis. In this study, the effects of FMOD on angiogenesis under both uninjured and wounded scenarios were investigated.

### Materials and Methods

### In ovo chick embryo chorioallantoic membrane (CAM) assay

The *in ovo* CAM assay was performed as previously described (23, 24). Fertilized chicken eggs (Charles River Labs, North Franklin, CT) were incubated at 37 °C and 60% relative humidity in an egg incubator. On day 3, 5 ml albumin was withdrawn from the pointed end of the egg. Rectangle windows were cut into the shell as a portal of access for the CAM. On day 10, 2.0 mg/ml FMOD in 30 µl 1:3-diluted growth-factor-reduced Matrigel^{™} (BD Bioscience, Franklin Lakes, NJ) was loaded on an autoclaved 5 x 5-mm polyester mesh layer (grid size: 530 µm; Component Supply Company, Fort Meade, FL) and incubated for 45 min at 37 °C for gel formation before transplantation onto the CAM. A non-FMOD phosphate buffered saline (PBS) control was transplanted onto the same CAM with a 1 cm distance. On day 13, CAMs were excised and photographed. The capillary area density directly under the mesh was measured by ImageJ (NIH, Bethesda, MD) (25).

### Matrigel^{™} plug Assay

400 µl of growth-factor-reduced MatrigelTM containing 0 or 4.0 mg/ml FMOD was subcutaneously injected into the abdomen of adult 129/sv male mice, which were harvested with the overlying skin 14 days post-injection (26).

### Wound generation

Four (per adult male 129/sv mouse) or six (per adult male Sprague-Dawley rat) full thickness, 10 mm x 3 mm skin ellipses with the underlying *panniculus carnosus* muscle were excised from each animal. All wounds were separated by at least 2 cm to minimize adjacent wound effects. Each open wound edge was injected with 25 µl PBS or 0.4 mg/ml recombinant human FMOD in PBS (25 µl x 2 edges = 50 µl/wound) before being primarily closed. Sutures were removed at day 7 post-injury, and wounds were harvested at 14 days post-injury. Tissues were bisected centrally for histology or gene expression analysis (3, 4, 14, 16).

### Histology and immunohistochemistry (IHC) staining

After fixation in 4% paraformaldehyde, samples were dehydrated, paraffin- embedded, and sectioned at 5-µm increments for Hematoxylin and Eosin (H&E), Picrosirius Red (PSR), and IHC staining (3, 4). PSR-coupled polarized light microscopy (PSR-PLM) was used to identify the wound area (3). Blood vessels were identified and quantitated by von Willebrand Factor (vWF; (Abcam Inc.,Cambridge, MA).

### Gene expression assay

RNA was isolated using RNeasy^{®} Mini Kit with DNase treatment (Qiagen) (3, 16). 1.0 µg mouse RNA was used for reverse transcription with iScriptTM Reverse Transcription Supermix for RT-qPCR (Bio-Rad Laboratories, Hercules, CA). Quantitative RT-PCR (qRT-PCR) was performed with TaqMan^{®} Gene Expression Assays (Life Technologies) and SsoFastTM Probes Supermix with ROX (Bio-Rad Laboratories) on a 7300 Real-Time PCR system (Life Technologies). Meanwhile, 2.5 µg RNA isolated from adult rat wounds was injected into RT² First Strand Kit (Qiagen) for reverse transcription. qRT-PCR was performed in a 96-well format of rat wound healing RT² PCR Array (Qiagen) according to the manufacturer's protocol. Three different cDNA templates were tested. Concomitant *glyceraldehyde 3-phosphate dehydrogenase (gapdh)* was used as a housekeeping standard. Data analysis was achieved by the manufacturer's online services (http://pcrdataanalysis.sabiosciences.com/pcr/arrayanalysis.php).

### Cell culture

Passages 3-6 human umbilical vein endothelial cells (HUVECs) were cultured in Medium 200PRF supplied with Low Serum Growth Supplement according to manufacturer instruction (Life Technologies).

### Tube-like structure (TLS) formation analysis

Technologies Endothelial Tube Formation Assay protocol provided by Life Technologies (http://www.lifetechnologies.com/us/en/home/references/protocols/cell-and-tissue-analysis/cell-profilteration-assay-protocols/angiogenesis-protocols/endothelial-cell-tube-formation-assay.html) was used to assay TLS *in vitro.* Briefly, a 24-well plate was coated with 100 µl/well reduced growth factor basement membrane matrix for 1 h at 37 °C before being seeded with 2.5 × 10⁴ HUVECs in Medium 200PRF supplied with different doses of FMOD. Five images per well and four wells per treatment were documented after 4 h by using an Olympus fluorescent microscope (Center Valley, PA). Images were assessed by recording dimensional and topological analyses with Image J (http://image.bio.methods.free.fr/ImageJ/7Angiogenesis-Analyzer-for-ImageJ.html&lang=en#outil_sommaire_0).

### Cell invasion assay

Cell invasion assay was performed in 24-well tissue culture plates using HTS Fluoroblok inserts with 8 µm pore size Fluorescence Blocking PET track-etched membranes (BD Bioscience). The upper surfaces of the inserts were coated with 100 µl of 2 mg/ml reduced growth factor basement membrane matrix (Geltrex^{®}; Life Technologies) and placed into 24-well tissue culture plates containing 750 µl medium. 2.5 × 10⁴ HUVECs in 500 µl medium with different doses of FMOD were added to each insert chamber and allowed to invade toward the underside of the membrane for 24 h. Non-invading cells were removed by wiping the upper side of the membrane with a cotton swab. Invaded cells were fixed and stained with 0.4 mg/ml 4',6-diamino-2-phenlindole (DAPI; Sigma-Aldrich, St. Louis, MO) before counting (3).

### Statistical analysis

Statistical significance was performed by OriginPro 8 (Origin Lab Corp., Northampton, MA), including one-way ANOVA, paired t-test, two-sample t-test, and Mann-Whitney analyses. P-values less than 0.05 were considered statistically significant.

### Results

FMOD promotes vascularization in uninjured scenarios FMOD administrated CAMs showed a 1.5-times greater proportion of blood vessels with large diameters than the PBS control (Fig. 1), confirming that FMOD promotes vasculogenesis during development. Since angiogenesis in adults may differ in important ways from the process during development (27), a pre-documented Matrigel^{™} plug assay (26) was used to confirm the pro-angiogenic action of FMOD *in vivo.* FMOD markedly elevated angiogenesis in Matrigel^{™} plugs subcutaneously implanted in adult mice, whose capillary densities were 4- fold that of non-FMOD plugs (Fig. 8). Thus, FMOD is a pro-angiogenic factor in uninjured scenarios.

FMOD is important for angiogenesis during wound healing in agreement with our previous studies at day 7 post-injury (3), vascular generation in adult *fmod*^{*-*/*-*} mouse skin wounds at day 14 post-injury was diminished by approximately 50% as compared to the age-matched WT wounds (Fig. 2). On the contrary, exogenous FMOD administration restored vascularity of *fmod*^{*-*/*-*} wounds to the same level as that of FMOD-treated WT wounds, further signifying that FMOD-deficiency was responsible for the reduced angiogenesis in *fmod*^{*-*/*-*}mouse wounds (Fig. 2). Additionally, capillary density of FMOD-treated adult WT mouse skin wounds was approximately 2.6-times greater than that of PBS-control groups (Fig. 2). This is in agreement with the finding that FMOD administration into an established adult rat cutaneous wound model causes a significant increase in wound vascularity (Fig. 3). Therefore, these results strongly endorse our hypothesis that FMOD is angiogenic in both uninjured and wounded scenarios.

### FMOD broadly enhances the transcription of angiogenic genes and impedes the expression of angiostatic genes

Double-transgenic mice overexpressing vascular endothelial growth factor (Vegf) and angiopoietin 1 (Angpt1) in skin showed a greater quantity and size of blood vessels (28). Vegf is massively produced by the epidermis during wound healing and has strong stimulating effects on angiogenesis via enhancement of microvascular permeability and stimulation of EC proliferation and migration (29- 33). There was no meaningful difference in *Vegf* expression between adult WT and *fmod*^{*-*/*-*} mouse unwounded skin tissues; however, *vegf* levels in WT wounds significantly increased at day 7 and 14 post-injury (Fig. 4, left). In contrast, *vegf* expression stayed at consistently low levels in *fmod*^{*-*/*-*} wounds throughout the entire 14-day wound healing period (Fig. 4, left). Meanwhile, FMOD significantly stimulated *vegf* expression in both WT and *fmod*^{*-*/*-*} adult mouse wounds (Fig. 4, left). Like Vegf, Angpt1 is highly specific for vascular endothelium. Secreted by pericytes, Angpt1 is required for EC survival and proliferation and for vessel maturation (28, 32, 34). Although no considerable difference in *angpt1* expression in unwounded skin tissues was observed between adult WT and *fmod*^{*-*/*-*} mice, transcription levels of *angpt1* were significantly lower in *fmod*^{*-*/*-*} wounds after wound closure compared to that of age-matched WT mouse wounds (Fig. 4, right). Interestingly, FMOD treated WT and *fmod*^{*-*/*-*} adult mouse wounds had similar *vegf* and *angpt1* levels at day 14 post-injury (Fig. 4), which was correlated to their similar wound capillary densities (Fig. 2). Considering the fact that *fmod*^{*-*/*-*} wounds have decreased vascularity which can be rescued by exogenous FMOD administration, these data are highly associated with Vegf's critical angiogenic function during granulation tissue formation and Angpt1's important mediation of vessel remodeling and maturation (28, 34-36).

Numerous angiogenic and angiostatic factors have been identified in the past (37, 38). In order to further enrich our knowledge of how FMOD affects angiogenesis-related genes during wound healing, a RT² PCR Array for rat wound healing (Qiagen, Valencia, CA) was employed for high-throughput gene expression analysis in an adult rat cutaneous wound model. As seen in the adult mouse data shown above, FMOD administration elevated both *angpt1* and *vegf* expression (Fig. 5). Moreover, FMOD not only upregulated the expression of *angpt1* and *vegf,* but also upregulated expression of other angiogenic genes such as *tgfα*[which stimulates chemotactic response, proliferation, and Vegf expression of ECs (39-41)], *fibroblast growth factor (fgf)2* [which induces EC proliferation, migration, and Vegf secretion (32, 42)], *platelet-derived growth factor (pdgf)*-*α*[which escorts connective tissue cells (such as fibroblasts and mast cells) into the wound area to produce angiogenic factors, and enhances angiogenic effects of Vegf and Fgf2 (43-46)], and *colony stimulation factor (csf)3* [which recruits monocytes to trigger the synthesis of angiogenic cytokines (33)] (Fig. 5). On the other hand, FMOD reduced the levels of angiostatic genes including *interferon (ifn) γ* [which inhibits EC growth and Vegf expression (47-49) and blocks capillary growth induced by Fgf and Pdgf (50)], *tgfβ1* [which hinders activation of differentiated ECs for sprouting and thus maintains endothelial quiescence (51)], and *plasminogen* [*plg;* which inhibits EC proliferation (52) and their response to Fgf and Vegf (53)] after wound closure (Fig. 5). Therefore, FMOD endorsed an angiogenesis-favoring gene expression network in adult rodent wound models.

### FMOD prompts EC tube-like structure (TLS) formation in vitro

To explore the direct effects of FMOD on EC spouting, the initial step of angiogenesis (21, 54), primary human umbilical vein endothelial cells (HUVECs) were seeded in Geltrex^{®} matrix (Life Technologies, Grand Island, NY), which contains laminin, collagen IV, entactin, and heparin sulfate proteoglycans to model a wound healing angiogenic situation. HUVECs spontaneously acquired elongated morphology and formed a capillary network in the gel, clearly visible by 3 hours post-seeding (Fig. 6, above). A broad range of FMOD (10-250 µg/ml) markedly enhanced HUVEC TLS formation and subsequently established polygon structures referred to as complex meshes (Fig. 6, above). Quantitative analyses demonstrated that FMOD significantly increased both dimensional (total length of cellular TLS network per area) and topological parameters (number of junctions, branches, and meshes per area) (Fig. 6, below) of HUVEC TLSs. In agreement with previous studies which revealed the positive relationship between EC migration and polygon structure formation (55), we found that FMOD significantly stimulated HUVEC invasion through the Geltrex^{®} matrix *in vitro* (Fig. 7). Therefore, FMOD exhibits its angiogenic function, at least partially, via promotion of EC migration/invasion.

Figure 8 shows Matrigel^{™} plugs subcutaneously injected into the abdomen of adult 129/sv male mouse. H&E staining (about) is shown with IHC staining against vWF (center) which was used to identify and quantitate blood vessels (below). Blood vessels are indicated with red arrowheads. FMOD: 4.0 mg/ml x 400 µl/plug. Significant differences compared by Mann-Whitney analysis (P < 0.05) are marked with asterisks (N = 5). Bar = 200 µm.

Figure 9 shows H&E staining and PSR-PLM demonstrate of adult mouse cutaneous wounds (outlined by dashed lines) at day 14 post-injury. FMOD: 0.4 mg/ml x 50 µl/wounds. Bar = 200 µm.

Figure 10 shows H&E staining of adult rat cutaneous wounds at day 14 post injury. The wound area was outlined by dashed lines), while IHC staining areas were outlined by dashed boxes. FMOD: 0.4 mg/ml x 50 µl/wounds. Bar = 400 µm.

### Discussion

Angiogenesis, a process of neovascular formation from pre-existing blood vasculature by sprouting, splitting, and remodeling of the primitive vascular network, results from multiple signals acting on ECs regulated by diverse groups of growth factors and ECM molecules (32, 44, 54). Until now, most studies on angiogenesis focused on soluble factors such as Vegf and Fgf2 (30-33, 42). However, increasing reports reveal that cell-ECM interaction is also critical for EC growth, differentiation, apoptosis, and response to soluble growth factors (10, 56, 57). For instance, blockage of EC-ECM interactions inhibits neovascularization *in vivo* and TLS formation *in vitro* (58-60). These findings indicate that successful angiogenesis requires a dynamic temporally and spatially regulated interaction between ECs, angiogenic factors, and surrounding ECM molecules such as SLRPs (21, 22, 32).

SLRPs are a family of proteins, including decorin, lumican, and FMOD, that are present within the ECM of all tissues (4-10). Since recent studies have shown that SLRPs interact with a diversity of cell surface receptors, cytokines, growth factors, and other ECM components resulting in modulation of cell-ECM cross talk and multiple biological processes (10-15), the common functionalities of SLRPs are far beyond their structural functions in the ECM (10, 15, 61). Specifically, intensive studies present a controversial function of decorin in angiogenesis: decorin is angiogenic during development and normal wound healing but is anti-angiogenic during tumor angiogenesis due to its ability to interfere with thrombospondin-1, suppress endogenous tumor Vegf production, and evoke stabilization of pericellular fibrillar matrix (10). Additionally, Niewiarowska et al. revealed that lumican inhibits angiogenesis by reducing proteolytic activity of ECs (62). However, unlike decorin and lumican, our current study revealed that FMOD is an angiogenic ECM molecule. Although FMOD and lumican present close homology and share the same binding region on type I collagen (63-65), their diverse influences on angiogenesis as well as epithelial migration (3, 16, 66) further support the hypothesis that FMOD and lumican do not appear to be functionally redundant, especially during cutaneous wound healing.

In this study, we demonstrated that not only did FMOD markedly enhance vasculogenesis during development, as documented by the *in ovo* CAM assay, but it also significantly stimulated angiogenesis as evidenced by the Matrigel^{™} plug assay as well as capillary density measurements in adult rodent cutaneous wound models. Additionally, impaired wound angiogenesis in *fmod*^{*-*/*-*} mice could be restored by exogenous FMOD administration. At the cellular level, we confirmed that FMOD boosted HUVEC migration/invasion and TLS formation *in vitro.* Our previous studies also found that, without considerable influence on EC proliferation, FMOD promoted EC cell adhesion, spreading, and actin stress fiber formation for vascularization *in vitro* (24). Thus, FMOD is an angiogenic ECM molecule that directly modulates EC behaviors. In addition to ECs, mural cells (such as fibroblasts and pericytes) and inflammatory cells (such as monocytes and mast cells) also contribute to wound angiogenesis (54, 67). By stimulating expression of various angiogenic factors including *angpt1, vegf, tgfα, fgf2, pdgfα,* and *csf3,* FMOD also activated these angiogenesis-related cells *in vivo* during the wound healing process. In contrast, Ifnyand Plg are anti-angiogenic, pro- inflammation molecules involved in wound healing (47-50, 52, 53, 68, 69). Additionally, Plg in particular also plays an important role in re-epithelialization, since keratinocyte migration over the wound is delayed in Plg-deficient mice (70).

In this study, FMOD administration reduced *ifnγ* and *plg* levels and increased angiogenesis in adult rodent wounds, which is highly correlated with our previous observation that cutaneous wounds of *fmod*^{*-*/*-*} mice exhibited extended inflammation, elevated epithelial migration, and insufficient angiogenesis (3, 16). Moreover, Tgfβ1, a multipotent growth factor that regulates wound healing, promotes endothelial cell differentiation in a Vegf-independent manner at early stages of development, but inhibits sprouting angiogenesis in differentiated ECs (51). Thus, lower *tgfβ1* transcription after wound closure could also contribute to enhanced angiogenesis in FMOD-treated wounds. Consistent with previous studies(24, 71), FMOD administration induced a pro-angiogenic microenvironment for wound healing *in vivo* by stimulating angiogenic factors and reducing angiostatic molecules.

In summary, as one of the pioneer groups investigating the influence of SLRPs on wound healing, we elucidated the angiogenic properties of FMOD in wounded scenarios, which function at least partially by promoting EC activation and infiltration in the wound area. While translation from the pre-clinical to the clinical setting can be difficult due to an increased number of external factors such as bacterial inhibition, taken together, current studies suggest that FMOD maintains the potential to be an attractive therapeutic candidate for wound management, especially for patients suffering from impaired wound healing due to aberrant cellular infiltration and insufficient angiogenesis, such as in the cases of diabetic wounds (72-74).

### References

1. Broughton, G., 2nd, Janis, J. E., Attinger, C. E. The basic science of wound healing. Plast Reconstr Surg 2006;117:12S-34S.
2. Diegelmann, R. F., Evans, M. C. Wound healing: an overview of acute, fibrotic and delayed healing. Frontiers in bioscience : a journal and virtual library 2004;9:283-289.
3. Zheng, Z., Nguyen, C., Zhang, X. L., et al. Delayed Wound Closure in Fibromodulin-Deficient Mice Is Associated with Increased TGF-beta 3 Signaling. J Invest Dermatol 2011;131:769-778.
4. Khorasani, H., Zheng, Z., Nguyen, C., et al. A Quantitative Approach to Scar Analysis. American Journal of Pathology 2011;178:621-628.
5. Ezura, Y., Chakravarti, S., Oldberg, A., Chervoneva, I., Birk, D. E. Differential expression of lumican and fibromodulin regulate collagen fibrillogenesis in developing mouse tendons. J Cell Biol 2000;151:779-787.
6. Font, B., Eichenberger, D., Goldschmidt, D., Boutillon, M. M., Hulmes, D. J. Structural requirements for fibromodulin binding to collagen and the control of type I collagen fibrillogenesis--critical roles for disulphide bonding and the C- terminal region. European journal of biochemistry / FEBS 1998;254:580-587.
7. Goldberg, M., Septier, D., Oldberg, A., Young, M. F., Ameye, L. G. Fibromodulin-deficient mice display impaired collagen fibrillogenesis in predentin as well as altered dentin mineralization and enamel formation. J Histochem Cytochem 2006;54:525-537.
8. Oldberg, A., Kalamajski, S., Salnikov, A. V., et al. Collagen-binding proteoglycan fibromodulin can determine stroma matrix structure and fluid balance in experimental carcinoma. Proceedings of the National Academy of Sciences of the United States of America 2007;104:13966-13971.
9. Svensson, L., Aszodi, A., Reinholt, F., Heinegard, D., Oldberg, A. Fibromodulin-null mice have abnormal collagen fibrils, tissue organization, and altered lumican deposition in tendon. - J Biol Chem 1993 Dec 15;268(35):26634- 44 1999;274:9636-9647.
10. Iozzo, R. V., Goldoni, S., Berendsen, A. D., Young, M. F. Small Leucine- Rich Proteoglycans: The Extracellular Matrix: an Overview. In R. P. Mecham ed.: Springer Berlin Heidelberg; 2011:197-231.
11. Bi, Y., Ehirchiou, D., Kilts, T. M., et al. Identification of tendon stem/progenitor cells and the role of the extracellular matrix in their niche. Nat Med 2007;13:1219-1227.
12. Zheng, Z., Jian, J., Zhang, X. L., et al. Reprogramming of human fibroblasts into multipotent cells with a single ECM proteoglycan, fibromodulin. Biomaterials 2012;33:5821-5831.
13. Soo, C., Beanes, S., Dang, C., Zhang, X., Ting, K. Fibromodulin, a TGF-β modulator, promotes scarless fetal repair. Surgical Forum 2001:578-581.
14. Soo, C., Hu, F. Y., Zhang, X., et al. Differential expression of fibromodulin, a transforming growth factor-beta modulator, in fetal skin development and scarless repair. Am J Pathol 2000;157:423-433. 15. Merline, R., Schaefer, R. M., Schaefer, L. The matricellular functions of small leucine-rich proteoglycans (SLRPs). J Cell Commun Signal 2009;3:323-335.
16. Zheng, Z., Lee, K. S., Zhang, X., et al. Fibromodulin-deficiency Alters Temporospatial Expression Patterns of Transforming Growth Factor-□Ligands and Receptors during Adult Mouse Skin Wound Healing. PloS one 2014;9:e90817.
17. Stoff, A., Rivera, A. A., Mathis, J. M., et al. Effect of adenoviral mediated overexpression of fibromodulin on human dermal fibroblasts and scar formation in full-thickness incisional wounds. J Mol Med 2007;85:481-496.
18. Zheng, Z., Zara, J. N., Nguyen, V. T., et al. Fibromodulin, a TGF-beta modulator, inhibits scar formation and increases tensile strength. J Am Coll Surgeons 2011;213:S99.
19. Zheng, Z., Yin, W., Zara, J., et al. The role of fibromodulin in fibroblast migration and enhanced wound closure. J Am Coll Surgeons 2010;211:S127- S127.
20. Arnold, F., West, D. C. Angiogenesis in wound healing. Pharmacol Ther 1991;52:407-422.
21. Tonnesen, M. G., Feng, X., Clark, R. A. Angiogenesis in wound healing. The journal of investigative dermatology Symposium proceedings / the Society for Investigative Dermatology, Inc [and] European Society for Dermatological Research 2000;5:40-46.
22. Kleinman, H. K., Malinda, K. M. Role of Angiogenesis in Wound Healing. In S. A. Mousa ed., Angiogenesis Inhibitors and Stimulators: Potential Therapeutic Implications: Landes Bioscience; 2000.
23. West, D. C., Thompson, W. D., Sells, P. G., Burbridge, M. F. Angiogenesis assays using chick chorioallantoic membrane. In M. J.C. ed., Methods in Molecular Medicine, Vol. 46: Angiogenesis Protocols. Tortowa, NJ: Humana Press Inc.; 2011.
24. Jian, J., Zheng, Z., Zhang, K., et al. Fibromodulin promoted in vitro and in vivo angiogenesis. Biochemical and Biophysical Research Communication 2013;436.
25. Maragoudakis, M. E., Haralabopoulos, G. C., Tsopanoglou, N. E., Pipilisynetos, E. Validation of Collagenous Protein-Synthesis as an Index for Angiogenesis with the Use of Morphological Methods. Microvascular research 1995;50:215-222.
26. Ouchi, N., Kobayashi, H., Kihara, S., et al. Adiponectin stimulates angiogenesis by promoting cross-talk between AMP-activated protein kinase and Akt signaling in endothelial cells. Journal of Biological Chemistry 2004;279:1304- 1309.
27. Davis, G. E., Senger, D. R. Endothelial extracellular matrix - Biosynthesis, remodeling, and functions during vascular morphogenesis and neovessel stabilization. Circulation research 2005;97:1093-1107.
28. Thurston, G., Suri, C., Smith, K., et al. Leakage-resistant blood vessels in mice transgenically overexpressing angiopoietin-1. Science 1999;286:2511-2514.
29. Dvorak, H. F., Brown, L. F., Detmar, M., Dvorak, A. M. Vascular permeability factor/vascular endothelial growth factor, microvascular hyperpermeability, and angiogenesis. Am JPathol 1995;146:1029-1039.
30. Coultas, L., Chawengsaksophak, K., Rossant, J. Endothelial cells and VEGF in vascular development. Nature 2005;438:937-945.
31. Olsson, A. K., Dimberg, A., Kreuger, J., Claesson-Welsh, L. VEGF receptor signalling - in control of vascular function. Nature reviews Molecular cell biology 2006;7:359-371.
32. Katoh, M. Therapeutics targeting angiogenesis: genetics and epigenetics, extracellular miRNAs and signaling networks (Review). International journal of molecular medicine 2013;32:763-767.
33. Lu, J. W., Pompili, V. J., Das, H. Neovascularization and Hematopoietic Stem Cells. Cell Biochem Biophys 2013;67:235-245.
34. Fagiani, E., Christofori, G. Angiopoietins in angiogenesis. Cancer Lett 2013;328:18-26.
35. Brown, L. F., Yeo, K. T., Berse, B., et al. Expression Of Vascular- Permeability Factor (Vascular Endothelial Growth-Factor) by Epidermal- Keratinocytes during Wound-Healing. J Exp Med 1992;176:1375-1379.
36. Nissen, N. N., Polverini, P. J., Koch, A. E., Volin, M. V., Gamelli, R. L., DiPietro, L. A. Vascular endothelial growth factor mediates angiogenic activity during the proliferative phase of wound healing. American Journal Of Pathology 1998;152:1445-1452.
37. Hanahan, D. Signaling vascular morphogenesis and maintenance. Science 1997;277:48-50.
38. Hanahan, D., Folkman, J. Patterns and emerging mechanisms of the angiogenic switch during tumorigenesis. Cell 1996;86:353-364.
39. Schreiber, A. B., Winkler, M. E., Derynck, R. Transforming Growth Factor- Alpha - a More Potent Angiogenic Mediator Than Epidermal Growth-Factor. Science 1986;232:1250-1253.
40. Grotendorst, G. R., Soma, Y., Takehara, K., Charette, M. EGF and TGF- alpha are potent chemoattractants for endothelial cells and EGF-like peptides are present at sites of tissue regeneration. J Cell Physiol 1989;139:617-623.
41. Gille, J., Swerlick, R. A., Caughman, S. W. Transforming growth factor- alpha-induced transcriptional activation of the vascular permeability factor (VPF/VEGF) gene requires AP-2-dependent DNA binding and transactivation. - EMBO J 1989 Sep;8(9):2601-4 1997;16:750-759.
42. Presta, M., Dell'Era, P., Mitola, S., Moroni, E., Ronca, R., Rusnati, M. Fibroblast growth factor/fibroblast growth factor receptor system in angiogenesis. Cytokine Growth Factor Rev 2005;16:159-178.
43. Cochran, B. H., Reffel, A. C., Stiles, C. D. Molecular cloning of gene sequences regulated by platelet-derived growth factor. Cell 1983;33:939-947.
44. Eming, S. A., Medalie, D. A., Tompkins, R. G., Yarmush, M. L., Morgan, J. R. Genetically modified human keratinocytes overexpressing PDGF-A enhance the performance of a composite skin graft. Human gene therapy 1998;9:529-539.
45. Gruber, B. L., Marchese, M. J., Kew, R. Angiogenic factors stimulate mast-cell migration. Blood 1995;86:2488-2493.
46. Marx, M., Perlmutter, R. A., Madri, J. A. Modulation of platelet-derived growth factor receptor expression in microvascular endothelial cells during in vitro angiogenesis. J Clin Invest 1994;93:131-139.
47. Friesel, R., Komoriya, A., Maciag, T. Inhibition of endothelial cell proliferation by gamma-interferon. J Cell Biol 1987;104:689-696.
48. Tsuruoka, N., Sugiyama, M., Tawaragi, Y., et al. Inhibition of in vitro angiogenesis by lymphotoxin and interferon-gamma. Biochemical and biophysical research communications 1988;155:429-435.
49. Kommineni, V. K., Nagineni, C. N., William, A., Detrick, B., Hooks, J. J. IFN-gamma acts as anti-angiogenic cytokine in the human cornea by regulating the expression of VEGF-A and sVEGF-R1. Biochemical and biophysical research communications 2008;374:479-484.
50. Sato, N., Nariuchi, H., Tsuruoka, N., et al. Actions of TNF and IFN-gamma on angiogenesis in vitro. J Invest Dermatol 1990;95:85S-89S. 51. Mallet, C., Vittet, D., Feige, J. J., Badly, S. TGFbetal induces vasculogenesis and inhibits angiogenic sprouting in an embryonic stem cell differentiation model: respective contribution of ALK1 and ALK5. Stem Cells 2006;24:2420-2427.
52. O'Reilly, M. S. Angiostatin: an endogenous inhibitor of angiogenesis and of tumor growth. Exs 1997;79:273-294.
53. Oh, C. W., Hoover-Plow, J., Plow, E. F. The role of plasminogen in angiogenesis in vivo. Journal of thrombosis and haemostasis : JTH 2003;1:1683- 1687.
54. Karamysheva, A. F. Mechanisms of angiogenesis. Biochemistry-Moscow+ 2008;73:751-762.
55. Aranda, E., Owen, G. I. A semi-quantitative assay to screen for angiogenic compounds and compounds with angiogenic potential using the EA.hy926 endothelial cell line. Biological research 2009;42:377-389.
56. Sottile, J. Regulation of angiogenesis by extracellular matrix. Bba-Rev Cancer 2004;1654:13-22.
57. Krishnan, L., Hoying, J. B., Nguyen, H., Song, H., Weiss, J. A. Interaction of angiogenic microvessels with the extracellular matrix. Am J Physiol-Heart C 2007;293:H3650-H3658.
58. Britsch, S., Christ, B., Jacob, H. J. The influence of cell-matrix interactions on the development of quail chorioallantoic vascular system. Anatomy and embryology 1989;180:479-484.
59. Belotti, D., Foglieni, C., Resovi, A., Giavazzi, R., Taraboletti, G. Targeting angiogenesis with compounds from the extracellular matrix. Int J Biochem Cell B 2011;43:1674-1685.
60. Stromblad, S., Cheresh, D. A. Cell adhesion and angiogenesis. Trends Cell Biol 1996;6:462-468.
61. Iozzo, R. V., Schaefer, L. Proteoglycans in health and disease: novel regulatory signaling mechanisms evoked by the small leucine-rich proteoglycans. Febs J 2010;277:3864-3875.
62. Niewiarowska, J., Brezillon, S., Sacewicz-Hofman, I., et al. Lumican inhibits angiogenesis by interfering with alpha2beta1 receptor activity and downregulating MMP-14 expression. Thrombosis research 2011;128:452-457.
63. Matheson, S., Larjava, H., Hakkinen, L. Distinctive localization and function for lumican, fibromodulin and decorin to regulate collagen fibril organization in periodontal tissues. J Periodontal Res 2005;40:312-324.
64. Kalamajski, S., Oldberg, A. Homologous Sequence in Lumican and Fibromodulin Leucine-rich Repeat 5-7 Competes for Collagen Binding. Journal Of Biological Chemistry 2009;284:534-539.
65. Svensson, L., Narlid, I., Oldberg, A. Fibromodulin and lumican bind to the same region on collagen type I fibrils. FEBS letters 2000;470:178-182.
66. Yamanaka, O., Yuan, Y., Coulson-Thomas, V. J., et al. Lumican Binds ALK5 to Promote Epithelium Wound Healing. PloS one 2013;8:e82730.
67. Imhof, B. A., Aurrand-Lions, M. Angiogenesis and inflammation face off. Nature Medicine 2006;12:171-172.
68. Maheswaran, V., Schmidt, Z., Koshova, O., Lunov, O., Syrovets, T., Simmet, T. Effects of Plasmin on Endothelial Cells. N-S Arch Pharmacol 2012;385:90-90.
69. Syrovets, T., Lunov, O., Simmet, T. Plasmin as a proinflammatory cell activator. J Leukocyte Biol 2012;92:509-519.
70. Romer, J., Bugge, T. H., Pyke, C., et al. Impaired wound healing in mice with a disrupted plasminogen gene. Nature Medicine 1996;2:287-292.
71. Adini, I., Ghosh, K., Adini, A., et al. Melanocyte-secreted fibromodulin promotes an angiogenic microenvironment. Journal Of Clinical Investigation 2014;124:425-436.
72. Gibran, N. S., Jang, Y. C., Isik, F. F., et al. Diminished neuropeptide levels contribute to the impaired cutaneous healing response associated with diabetes mellitus. Journal of Surgical Research 2002;108:122-128.
73. Falanga, V. Wound healing and its impairment in the diabetic foot. Lancet 2005;366:1736-1743.
74. Galiano, R. D., Tepper, O. M., Pelo, C. R., et al. Topical vascular endothelial growth factor accelerates diabetic wound healing through increased angiogenesis and by mobilizing and recruiting bone marrow-derived cells. American Journal of Pathology 2004;164:1935-1947.

### Example 2. Fibromodulin reduces scar formation in rodent and porcine cutaneous wound models

Cutaneous scars affect over 100 million patients annually, and are major concerns for those suffering from debilitating medical conditions. Unfortunately, the current methods of scar treatment and reduction are minimally effective or have undesirable side effects. By using fetal rodent cutaneous wound models, we demonstrated that fibromodulin (FMOD) is essential for scarless fetal-type repair. FMOD also exhibited potent anti-scarring effects in loss- and gain-of-function rodent models and increased wound tensile strength in adult rodent and two porcine models that simulate normal and hypertrophic human cutaneous repair. Instead of simply antagonizing, FMOD orchestrated transforming growth factor (TGF)-beta signaling in an isoform-specific and signal transduction-specific manner. Thus, FMOD induced fibroblast migration, differentiation, and contraction, which accelerated timely, wound closure, and inhibited fibrotic extracellular matrix expression, which promoted reduced scar formation. Moreover, FMOD stimulated interleukin 1b expression, a known accelerant of myofibroblast apoptosis and the principal cell type implicated in pathological scarring. Overall, FMOD drives cellular migration, differentiation, and contraction, as well as myofibroblast apoptosis through diverse signaling pathways to promote optimal repair. These findings strongly suggest the potential clinical utility of FMOD for prevention and treatment of human scarring such as hypertrophic scars, keloids, and other fibrotic conditions.

## Claims

1. A biocompatible device comprising a body structure and a wound-healing-enhancing agent in a wound-healing-enhancing effective amount,
the biocompatible device further comprising a coating, wherein the wound-healing enhancing agent is included in the coating,
the coating comprising microparticles and/or nanoparticles, wherein the wound-healing-enhancing agent is encapsulated into the microparticles and/or nanoparticles;
wherein the microparticles and/or nanoparticles are admixed with or encapsulated within a polymeric material matrix;
wherein the wound-healing-enhancing agent is fibromodulin (FMOD), an FMOD polypeptide, or an FMOD peptide;
wherein the FMOD, FMOD polypeptide, or FMOD peptide is modified by fusion to a polypeptide sequence or by attachment to a polymer; and
wherein the biocompatible device is a medical implant or cosmetic implant.

2. The biocompatible device of claim 1, further comprising a wound-healing agent.

3. A method of fabricating the biocompatible device of claim 1 or claim 2, which method comprises:
providing a body structure of the biocompatible device, and
forming the coating.

## Patentansprüche

1. Biokompatible Vorrichtung, aufweisend eine Körperstruktur und ein wundheilungsförderndes Mittel in einer wundheilungsfördernden wirksamen Menge,
wobei die biokompatible Vorrichtung ferner eine Beschichtung aufweist, wobei das wundheilungsfördernde Mittel in der Beschichtung enthalten ist,
wobei die Beschichtung Mikropartikel und/oder Nanopartikel aufweist, wobei das wundheilungsfördernde Mittel in den Mikropartikeln und/oder Nanopartikeln eingekapselt ist;
wobei die Mikropartikel und/oder Nanopartikel mit einer Polymermaterialmatrix vermischt oder darin eingekapselt sind;
wobei das wundheilungsfördernde Mittel Fibromodulin (FMOD), ein FMOD-Polypeptid oder ein FMOD-Peptid ist;
wobei das FMOD, FMOD-Polypeptid oder FMOD-Peptid durch Fusion mit einer Polypeptidsequenz oder durch Anlage an ein Polymer modifiziert ist; und
wobei die biokompatible Vorrichtung ein medizinisches Implantat oder ein kosmetisches Implantat ist.

2. Biokompatible Vorrichtung nach Anspruch 1, ferner aufweisend ein Wundheilungsmittel.

3. Verfahren zur Herstellung der biokompatiblen Vorrichtung nach Anspruch 1 oder Anspruch 2, welches Verfahren aufweist:
Bereitstellen einer Körperstruktur der biokompatiblen Vorrichtung, und Bilden der Beschichtung.

## Revendications

1. Dispositif biocompatible, comprenant une structure corporelle et un agent favorisant la cicatrisation des plaies dont la quantité est efficace pour favoriser la cicatrisation des plaies,
le dispositif biocompatible comprenant en outre un revêtement, dans lequel l'agent favorisant la cicatrisation des plaies est inclus dans le revêtement, le revêtement comprenant des microparticules et/ou des nanoparticules, dans lequel l'agent favorisant la cicatrisation des plaies est encapsulé dans les microparticules et/ou les nanoparticules ;
dans lequel les microparticules et/ou les nanoparticules sont mélangées ou encapsulées dans une matrice polymère ;
dans lequel l'agent favorisant la cicatrisation des plaies est la fibromoduline (FMOD), un polypeptide de la FMOD, ou un peptide de la FMOD ;
dans lequel la FMOD, le polypeptide de la FMOD, ou le peptide de la FMOD est modifié(e) par fusion en une séquence de polypeptides ou par fixation en un polymère ; et
dans lequel le dispositif biocompatible est un implant médical ou un implant cosmétique.

2. Dispositif biocompatible selon la revendication 1, comprenant en outre un agent de cicatrisation des plaies.

3. Procédé de fabrication du dispositif biocompatible de la revendication 1 ou 2, lequel procédé consiste à :
fournir une structure corporelle du dispositif biocompatible ; et
former le revêtement.
